(19) **Europäisches Patentamt**

**European Patent Office**

**Office européen des brevets**

(11) **EP 1 490 504 B1**

(12) **EUROPEAN PATENT SPECIFICATION**

(45) Date of publication and mention
of the grant of the patent:
**27.12.2006 Bulletin 2006/52**

(51) Int Cl.:
*C12Q 1/00* (2006.01)     *G01N 7/00* (2006.01)
*C12M 1/00* (2006.01)

(21) Application number: **03744312.4**

(22) Date of filing: **12.03.2003**

(86) International application number:
**PCT/DK2003/000153**

(87) International publication number:
**WO 2003/078653 (25.09.2003 Gazette 2003/39)**

(54) **METHOD AND APPARATUS FOR MONITORING A FERMENTATION PROCESS**

VERFAHREN UND VORRICHTUNG ZUR ÜBERWACHUNG EINES FERMENTATIONSPROZESSES

PROCEDE ET APPAREIL PERMETTANT DE SURVEILLER UN PROCEDE DE FERMENTATION

(84) Designated Contracting States:
**AT BE BG CH CY CZ DE DK EE ES FI FR GB GR HU IE IT LI LU MC NL PT RO SE SI SK TR**

(30) Priority: **18.03.2002 DK 200200421**

(43) Date of publication of application:
**29.12.2004 Bulletin 2004/53**

(73) Proprietor: **VINIBIOS APS**
**2100 Copenhagen (DK)**

(72) Inventor: **NIELSEN, Jan, Clair**
**DK-3390 Hundested (DK)**

(74) Representative: **HOEIBERG A/S**
**European Patent & Trademark Attorneys**
**Store Kongensgade 59A**
**1264 Copenhagen K (DK)**

(56) References cited:
**EP-A- 0 271 380          DE-B- 2 744 769**

• HORIUCHI JUN-ICHI ET AL: "Fuzzy-aided estimation of biological parameters based on material balances." JOURNAL OF FERMENTATION AND BIOENGINEERING, vol. 86, no. 1, 1998, pages 111-117, XP002243731 ISSN: 0922-338X

• WICK M ET AL: "Pressure measurement to evaluate ethanol or lactic acid production during glucose fermentation by yeast or heterofermentative bacteria in pure and mixed culture." APPLIED MICROBIOLOGY AND BIOTECHNOLOGY, vol. 56, no. 5-6, September 2001 (2001-09), pages 687-692, XP002243732 ISSN: 0175-7598

• CLAES J E ET AL: "Combining yield coefficients and exit-gas analysis for monitoring of the baker's yeast fed-batch fermentation." BIOPROCESS ENGINEERING, vol. 22, no. 3, March 2000 (2000-03), pages 195-200, XP002243733 ISSN: 0178-515X

• DATABASE BIOSIS [Online] BIOSCIENCES INFORMATION SERVICE, PHILADELPHIA, PA, US; 1988 EL HALOUI N ET AL: "ALCOHOLIC FERMENTATION IN WINEMAKING ON-LINE MEASUREMENT OF DENSITY AND CARBON DIOXIDE EVOLUTION" Database accession no. PREV198987058256 XP002243734 & JOURNAL OF FOOD ENGINEERING, vol. 8, no. 1, 1988, pages 17-30, ISSN: 0260-8774

• DATABASE BIOSIS [Online] BIOSCIENCES INFORMATION SERVICE, PHILADELPHIA, PA, US; 1994 NAM SOO WAN ET AL: "Indirect estimation of cell mass substrate concentration using a computer-coupled mass spectrometry." Database accession no. PREV199497237461 XP002243735 & JOURNAL OF FERMENTATION AND BIOENGINEERING, vol. 77, no. 3, 1994, pages 332-334, ISSN: 0922-338X

- DATABASE STN INTERNATIONAL [Online] File CAPLUS, CAPLUS accession no. 1954:36923, document no. 48:6612e-h; BOHN, R.T.: "Determination of reducing sugars in bread by biological methods" XP002243736
- DATABASE STN INTERNATIONAL [Online] File CAPLUS, CAPLUS accession no. 1956:7730, document no 50:7730; BERAN, KAREL ET AL: "New gasometric method for the determiantion fo sugars by fermentation in the presence of 2,4,dinitrophenol" XP002243737
- DATABASE WPI Section Ch, Week 197503 Derwent Publications Ltd., London, GB; Class D11, AN 1975-05131W XP002243738 & SU 418 798 A (BAKING INDS INST), 14 August 1974 (1974-08-14)

## Description

### Field of invention

The present invention relates to methods of determining the amount of a fermentable compound in a liquid, such as for example the amount of sugar or malic acid. Furthermore the invention relates to apparatuses and compositions useful in said methods.

### Background of invention

Fermentation processes are employed to produce a number of different foods and beverages, for example alcohol containing beverages such as beer and wine.

Wine production usually comprises at least two fermentation steps, alcohol fermentation wherein sugar is fermented to produce ethanol and $CO_2$ and the malolactic fermentation wherein malic acid is fermented to lactic acid and $CO_2$.

During the alcohol fermentation the yeast *Saccharomyces cerevisiae* converts the sugar in grape juice to alcohol. The first part of said fermentation is rapid, whereas the last part of the fermentation when around 1 to 20 g of sugar/l wine is left is a slow process. This process is often monitored by the wine producer by determining the amount of residual sugar in the wine. It is often desirable for the wine producer to let the wine ferment completely leaving essentially no residual sugar, partly to achieve the desired flavour and partly to avoid later yeast growth in the finished product.

The methods currently used by wine producers to determine residual sugar are technically and practically difficult to handle and not directly related to the amount of fermentable residual sugar in the wine.

A second important step in wine production is the malolactic fermentation. This fermentation process is undertaken by lactic acid bacteria and takes place just after alcohol fermentation. During the malolactic fermentation, lactic acid bacteria convert malic acid to lactic acid and $CO_2$. The conversion reduces the acidity of the wine and confers a more complete flavour.

It is of great importance to the wine producer to be able to monitor the malolactic fermentation, partly to ensure that it has started and partly to be able to stop all microbial activity, such as microbial growth in the wine once the malolactic fermentation is completed. Microbial activity is usually inhibited by sulphur treatment, which also protects the wine from oxidation.

The most common method to monitor the malolactic fermentation is by measuring the concentration of malic acid in the wine. Normally the starting concentration of malic acid is 2-8 g/l. When the malic acid content is 0-0.3 g/l the fermentation is considered complete and the wine producer may treat the wine with sulphur.

The methods presently used to measure malic acid in wine are very time consuming and expensive and all require educated technical personal.

Beer is made by fermentation of sugars extracted from malted barley, mainly maltose by yeast. For the brewer, the amount of maltose left in the beer is a useful parameter for monitoring the brewing process.

In the prior art various methods of determining $CO_2$ absorption/desorption from beverages has been described, such as manometric methods.

Bach et al., 1992 describes means of determining desorption of $CO_2$ from sparkling wine using a piece of zeolite. $CO_2$ desorption in relation to residuals sugar and $CO_2$ content is given. The document does not describe methods of determining the residual sugar content.

Rammert and Pahl, 1992 describes that absorption of $CO_2$ in beverages is dependent on i.a. alcohol and sucrose content. An equation is given that can be used to correct manometric determination of $CO_2$ in beverages. The document does not describe methods of determining the sucrose content.

In Official Methods of Analysis, 1984, a manometric method of determining $CO_2$ content in wine is described. The methods involves addition of NaOH, $NaHCO_3$ and $H_2PO_4$.

Miranda et al., 1997, describes inhibition of malolactic fermentation by i.a. glucose. The malolactic activity is monitored by measuring $CO_2$ release using a Warburg apparatus and a volumetric method. The document does not describe methods of determining malic acid content.

### Summary of invention

There exist an unmet need for fast and easy to handle methods of determining for example the amount of residual sugar or malic acid during production of alcoholic beverages such as wine.

The present invention describes novel methods for determining the amount of fermentable compounds in liquid. The methods are fast and easy to handle, allowing even non-technically trained personal to perform them. The methods take advantage of use of microbial organisms to convert fermentable compounds to gaseous products, the amount of which may be determined by for example manometric methods. Furthermore the invention describes apparatus and compositions for use in said methods.

Hence it is a first objective of the present invention to provide methods for determining the amount of at least one fermentable first compound in a liquid composition, said method comprising the steps of

    i) providing a liquid composition comprising said at least one fermentable first compound,

    ii) providing a test microbial organism capable of fermenting said at least one fermentable first compound,

iii) contacting said liquid composition with said test microbial organism under conditions allowing said test microbial organism to ferment essentially all of said at least one fermentable first compound,

iv) fermenting essentially all of said at least one fermentable first compound, wherein said fermentation results in the production of at least one assayable gaseous product in an amount directly correlatable with the amount of said at least one first fermentable compound present in said liquid composition,

v) determining the produced amount of said at least one assayable gaseous product,

vi) correlating the produced amount of said at least one assayable gaseous product with the amount of said at least one fermentable first compound, and thereby determining the amount of said at least one fermentable first compound.

The step of determining the produced amount of said at least one assayable gaseous product may in one embodiment of the invention involve the steps of

a) contacting said test microbial organism and said liquid composition in a confined space of an air tight container having a first pressure,
b) incubating said test microbial organism and said liquid composition for a predetermined period of time,
c) determining a second pressure in said confined space following said incubation, and
d) correlating said second pressure to the starting content of said at least one fermentable first compound present in said liquid composition, and thereby determining the produced amount of said at least one assayable gaseous product.

The methods of the present invention may be employed for controlling a fermentation process, said fermentation process comprising the steps of

i) providing a liquid composition comprising said at least one fermentable first compound,

ii) providing at least one first microbial organism capable of fermenting said at least one fermentable first compound,

iii) defining a predetermined fermentation parameter,

iv) incubating said liquid composition with said at least one first microbial organism under conditions allowing said microbial organism to ferment at least part of said at least one fermentable first compound,

v) obtaining from said liquid composition, after a predetermined time of incubation, a test sample comprising said at least one fermentable first compound,

vi) determining the amount of said at least one first fermentable compound according to the methods described herein above; and

vii) comparing the amount of said at least one fermentable first compound with said predetermined fermentation parameter, and

viii) controlling said fermentation process based on the comparison of the amount of said at least one fermentable first compound with said predetermined fermentation parameter.

In addition, the methods according to the present invention may furthermore comprise determining the amount of at least one fermentable second compound comprising the steps

i) defining a predetermined second fermentation parameter,

ii) obtaining a test sample from said liquid composition after a second predetermined time of incubation,

iii) adding to said test sample at least one test microbial organism capable of fermenting essentially all of said at least one fermentable second compound,

iv) fermenting essentially all of said at least one fermentable second compound, wherein said fermentation results in the production of at least one assayable gaseous product in an amount directly correlatable with the amount of said at least one second fermentable compound present in said liquid composition at the time of obtaining the test sample,

v) determining the produced amount of said at least one assayable gaseous product,

vi) correlating the produced amount of said at least one assayable gaseous product with the amount of said at least one fermentable second compound present in the test sample at the time of obtaining said sample, thereby determining the amount of said at least one fermentable second compound, and

vii) comparing the amount of said at least one fermentable second compound with said predetermined second fermentation parameter, and

viii) controlling said fermentation process based on the comparison of the amount of said at least one fermentable second compound with said predetermined second fermentation parameter.

It is a second objective of the present invention to provide an apparatus for determining the amount of at least one fermentable first compound in a liquid composition, wherein said apparatus comprises

a) a manometer comprising an injection needle for measuring the pressure within an air tight, test sample container comprising a confined space, wherein said manometer is calibrated to measure the amount of said at least one fermentable first compound; and
b) an air tight, test sample container, wherein said container is pressure stable and penetratable by said injection needle; and
c) a display capable of directly displaying the amount of said at least one fermentable first compound.

It is a third objective of the present invention to provide solid compositions for determining the amount of at least one fermentable compound in a liquid composition, said solid composition comprising a dried microbial organism capable of fermenting said fermentable compound; and

a) a buffer compound capable of buffering a solution to a pH in the range of from 4,0 to 8,0; or
b) a solid inhibitor of at least one metabolic pathway producing a gaseous product; or
c) a solid compound capable of inhibiting yeast metabolism, but not bacterial metabolism.

**Description of Drawings**

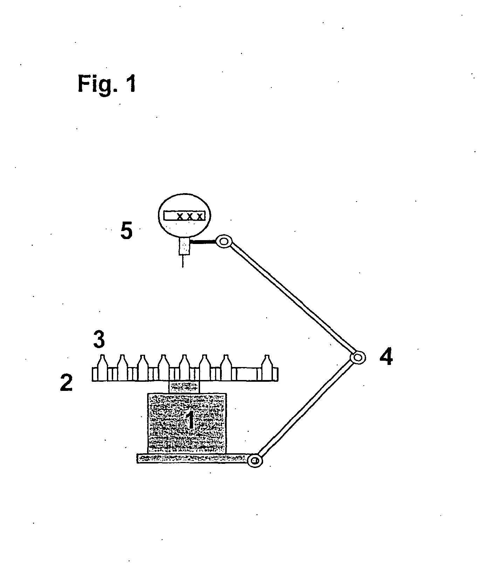

Figure 1 depicts an example of an apparatus for determining the amount of a fermentable compound in a liquid composition according to the present invention. 1) shaker; 2) solid support capable of fixating test sample containers; 3) test sample containers with membrane lids; 4) adjustable arm; 5) manometer mounted with a needle.

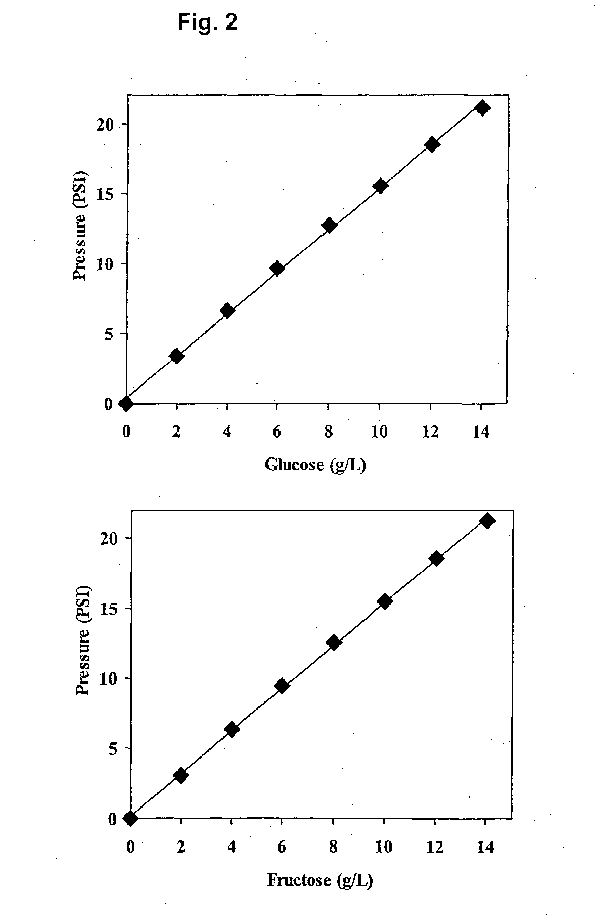

Figure 2 depicts a standard curves for glucose and fructose determination in red wine produced from Biotta grape juice as described in example 1. The results are based on fourfould determinations. The wine used had completed the alcoholic fermentation. Wine data: 12 % ethanol (v/v), pH 3.5, 0 g glucose/L, 0 g fructose/L.

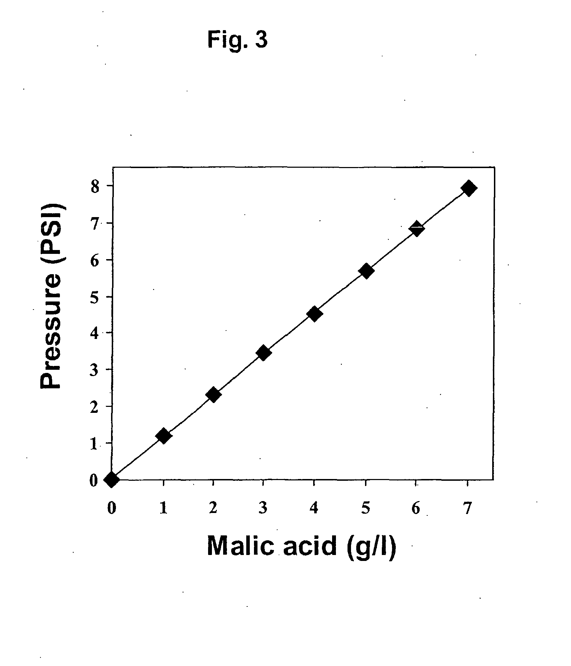

Figure 3 depicts a standard curve for L-malic acid determination in red wine produced from Biotta grape juice as described in example 2. The standard curve is based on fourfould determinations. The wine used had completed malolactic fermentation. Wine data: 12 % ethanol (v/v), pH 3.5, 0 g malic acid/l.

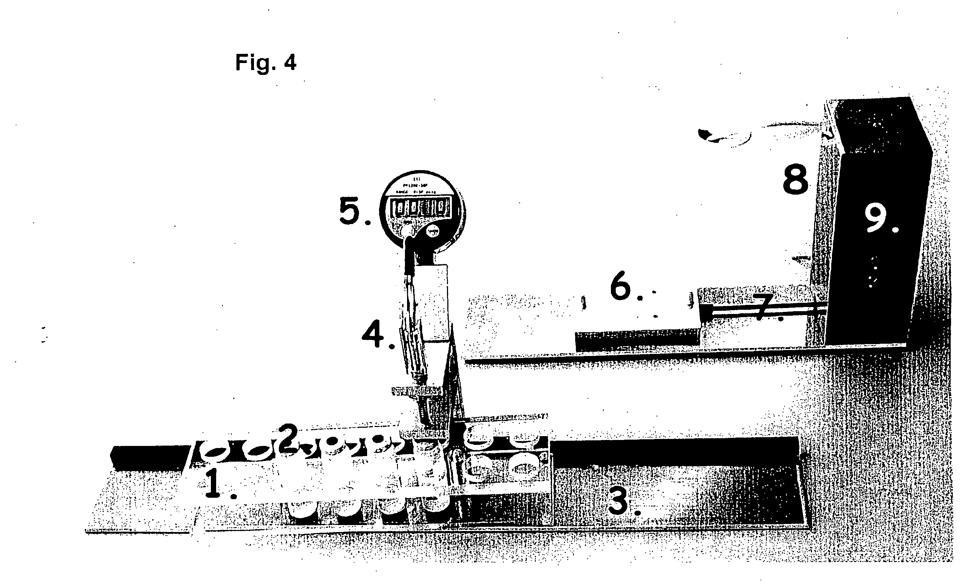

Figure 4 depicts an example of an apparatus useful for determination of malic acid and residual sugar in fermented products. Description of the different parts: 1: Rack for 16 glass containers. 2: Glass container with airtight crimp cap rubber lid (crimp cap with septa). 3: Plate whereupon the rack (1) can slide, 4: injection needle which can be pressed down through the rubber lid in the glass container (2) by use of the knob on the top. 5: Manometer which is connected to the injection needle (4) through a thin rubber tube. The manometer can measure the pressure in the glass container (2). The manometer may be calibrated to show the malic acid or residual sugar concentration directly. 6: Sledge whereupon the rack (1) can be placed. The sledge can be moved back and forward. 7: Arm connected to the sledge (6) and an electromotor. Can move the sledge back and forward. 8. Box containing the electromotor for the arm (7). 9: On/off and timer for the electromotor.

**Detailed description of the invention**

Fermentable compound

A fermentable compound according to the present invention is any compound capable of being fermented, i.e. broken down by an energy-generating process that does not require oxygen.

Frequently fermentation designates the conversion of a sugar to alcohol and carbon dioxide. However, fermentation may also be energy-generating breakdown of other organic molecules for example acids.

The present invention allows determining the amount of more than one fermentable compound, such as 2, for example 3, such as 4, for example 5, such as more than 5 fermentable compounds. Accordingly the amount of at least one first fermentable compound and the amount of at least one second fermentable compound and the amount of any further fermentable compounds may be determined according to the present methods.

The first fermentable compound, the second fermentable compound and any further fermentable compounds may individually be selected from the group consisting of sugars and acids.

In one embodiment of the present invention the sugar may be selected from the group consisting of glucose, fructose, sucrose and maltose and the acid may for example be malic acid.

In one preferred embodiment of the present invention the at least one fermentable first compound is a sugar, preferably a sugar selected from the group consisting of glucose, fructose, sucrose and maltose and the fermentable second compound is malic acid.

Gaseous product

A gaseous product according to the present invention is any product which is in the form of a gas at amble temperature and pressure.

I.e. the gaseous product is a gas at a temperature in the range from 0°C to 100°C, such as from 0°C to 50°C, such as from 5°C to 25°C, for example around room temperature, and at a pressure of 0,5 to 1,5 atm, preferably around 1 atm.

Examples of gaseous products include, but are not limited to $CO_2$, $CO$, $O_2$, $NO$ and $NO_2$. In one preferred embodiment of the present invention the gaseous product is $CO_2$.

The gaseous product according to the invention is preferably an assayable gaseous product, i.e. the amount of said gaseous product may be determined by an assay. The assay may be any assay, for example, the amount of gaseous product may be determined by determining pressure (see herein below).

Microbial organism

A microbial organism according to the present invention may be any microbial organism, such as for example bacteria, yeast or fungi.

The microbial organism to be employed with the present methods should be selected according to the nature of the fermentable compound desirable to determine, i.e. the microbial organism should be capable of fermenting the fermentable compound.

When the fermentable compound is a sugar, the microbial organism is preferably capable of fermenting sugar and thereby producing $CO_2$.

In one embodiment of the present invention the microbial organism is a yeast, in particular when the fermentable compound is a sugar the microbial organism is preferably yeast.

The yeast may for example belong to the *Saccharomyces* family. For example, the yeast may be selected from the group consisting of *Saccharomyces cerevisiae* and *Saccharomyces bayanus.*

Examples of commercially available yeast useful with the present methods include "Lalvin V1116" produced by Lallemand, Montreal, Canada and "Malteser Tørgær" from "De Danske Spritfabrikker", Grenaa, Denmark.

When the fermentable compound is malic acid, then the microbial organism is preferably capable of fermenting malic acid to lactic acid and thereby producing $CO_2$.

Accordingly, in one embodiment of the present invention the microbial organism may be a bacteria, in particular when the fermentable compound is an acid, then preferably the microbial organisms are bacteria.

The bacteria may for example be lactic acid bacteria, such as bacteria selected from the group of bacteria belonging to the *Oenococcus* family and the *Lactobacillus* family. Preferably, the bacteria are selected from the group consisting of *Oenococcus oeni* and *Lactobacillus plantarum.*

Examples of commercially available bacteria useful with the present methods include Viniflora oenos and Viniflora LP, both available from Chr. Hansen, Hørsholm, Denmark as well as EQ54 from Lallemand, Montréal, Canada.

In preferred embodiments of the present invention the microbial organisms are provided in a dried form in order to facilitate handling. The microbial organisms may be dried by any conventional drying method, which allows the microbial organism to resume metabolism and/or growth once the microbial organism is replaced in a aqueous environment.

For example the microbial organism may be dried by air drying, spray drying, freeze drying, drum drying or dried using a tray drier, ring-dryer, fluid-bed dryer or vacuum-band dryer. Preferably, the microbial organism may be freeze dried or the microbial organism may be spray dried.

The amount of microbial organism to be used with the methods according to the present invention depends on the amount of liquid composition, the nature of the liquid composition, the fermentable compound and the microbial organism to be used.

In general in the range of 5 mg to 1000 mg, such as 7 mg to 800 mg, for example 10 mg to 600 mg, such as 12 mg to 500 mg, for example 15 mg to 400 mg, such as 17 mg to 300 mg, for example 20 mg to 250 mg, such as 25 mg to 200 mg, for example 30 mg to 150 mg, such as 35 mg to 100 mg, for example 40 mg to 80 mg dry microbial organism is used per ml of liquid composition for the methods of determining a fermentable compound in said liquid composition.

In embodiments wherein the microbial organism is yeast, for example *Saccharomyces cerevisiae,* then preferably in the range of 5 mg to 500 mg, for example 10 mg to 400 mg, such as 12 mg to 300 mg, for example 15 mg to 200 mg, such as 17 mg to 150 mg, for example 20 mg to 100 mg, such as 25 mg to 75 mg, for example 30 mg to 50 mg, such as 35 mg to 45 mg, for example around 40 mg dry yeast is used per ml of liquid composition (for example fermented grape juice).

In embodiments wherein the microbial organism is bacteria, for example *Oenococcus oeni* or *Lactobacillus plantarum,* then preferably in the range of 5 mg to 1000 mg, such as 10 mg to 800 mg, for example 15 mg to 600 mg, such as 20 mg to 500 mg, for example 25 mg to 400 mg, such as 30 mg to 300 mg, for example 35 mg to 250 mg, such as 40 mg to 200 mg, for example 50 mg to 150 mg, such as 60 mg to 100 mg, for example 70 mg to 90 mg, such as from 75 mg to 85 mg, for example around 80 mg dry bacteria is used per ml of liquid composition (for example fermented grape juice).

Liquid composition

The present methods may be employed to measure fermentable compounds in any liquid composition. It is often desirable to determine the amount of fermentable compounds during the production of alcohol containing beverages and accordingly the liquid compositions may be any liquid, which is used in the production of alcohol containing beverages.

For example, the liquid composition may comprise fruit juice, partly fermented fruit juice or fermented fruit juice. It is also possible that the liquid composition essentially consists of or consists of fruit juice, partly fermented fruit juice or fermented fruit juice.

An example of a especially useful fruit juice is grape juice. Accordingly, the liquid composition may comprise or essentially consist of grape juice, partially fermented grape juice or fermented grape juice. Hence, in one embodiment the liquid composition may be wine or precursor of wine.

In another embodiment of the present invention the liquid composition comprises malt. Malt is the main ingredient in beer production and accordingly, the liquid may be beer or a precursor of beer. Beer may also comprise other ingredients than malt, for example the liquid may furthermore comprise hops. Malt according to the invention may be any kind of malt, such as malt produced from barley or wheat.

Buffer

In some embodiments of the present invention it is preferred that a buffer is added to the liquid composition. Alternatively, the composition comprising the microbial organism may comprise a buffer (see herein below). In particular, when the method comprises use of yeast to determine the amount of sugar in an acidic liquid a buffer may be added.

The pH of wine or partially fermented grape juice is around 3.0 to 3.8. Yeast metabolism is reduced at such low pH. Accordingly, it is preferred to add a buffer to a wine sample in order to improve yeast metabolism. This is of course true for any liquid composition to be used with the present invention, which has a pH lower than 4.5, preferably lower than 4.0.

Accordingly, in some embodiments of the present invention the methods furthermore comprise adding a predetermined amount of a buffer with the test microbial organism. The buffer may be any buffer capable of buffering a solution to a pH of more than 4,0. Preferably, the buffer is any buffer capable of buffering sample of wine or partially fermented grape juice to a pH of more than 4,0.

$CO_2$ has a pKa value around 6. Hence, $CO_2$ may be dissolved as bicarbonate in liquids with a pH higher than 6. If the gaseous product is $CO_2$, then it must be taken into consideration that a part of the $CO_2$ may be dissolved into a liquid composition with a pH of more than 6. Accordingly, it is preferred that the pH of the liquid solution is less than 6, when the gaseous compound is $CO_2$. However, it is possible to measure the amount of $CO_2$ even if the pH of the liquid is higher than 6. Then however the exact amount of $CO_2$ must be calculated

taking into consideration the amount of dissolved $CO_2$.

Hence, in one preferred embodiment of the present invention the buffer is capable of buffering a solution, for example a sample of wine or partially fermented grape juice to a pH in the range of from 4,0 to 8,0, for example from 4,0 to 7,5, such as from 4,0 to 7,0, for example from 4,0 to 6,5, such as from 4,0 to 6,0, for example from 4,5 to 6,0.

Preferably, the buffer may be any buffer which has a pKa value in the range from 4,0 to 8,0, such as from 4,0 to 7,5, for example from 4,0 to 7,0, such as from 4,0 to 6,5, for example from 4,0 to 6,0, such as from 4,0 to 5,5, for example from 4,5 to 5,5.

Examples of buffers useful with the present invention includes, but are not limited to phosphate buffers, citrate buffers, K biphthalate, acetate, dimethylglutaric acid, piperazine, tetraethylethylenediamine, tris maleate and dimethylaminoethylamine.

Accordingly, the buffer may be preferably selected from the group consisting of phosphate buffer and citrate buffer, more preferably the buffer is a citrate buffer. Phosphate buffers may for example be $K_2HPO_4$, $Na_2HPO_4$ and/or $KH_2PO_4$. Citrate buffer may for example be citrate dibasic sesquihydrate, $Na_2$Citrate or any other useful citrate buffer.

In preferred embodiments of the present invention the buffer is provided as solid matter, such as a dry powder. This allows long term storage of the buffer as well as long term storage of a composition comprising buffer mixed with a microbial organism, such as a dried microbial organism.

The predetermined amount of buffer, which should be used with the present invention, must be selected according to the amount and content of the liquid composition, in which the fermentable compound should be determined. Furthermore, the predetermined amount of buffer must be selected according to the nature of the buffer.

In general, the predetermined amount of buffer should be in range of 1 to 500 mg dry buffer per ml liquid composition. For example the predetermined amount of buffer may be in range of 2 to 450 mg, such as from 5 to 400 mg, for example from 5 to 350 mg, such as from 5 to 300 mg, for example from 5 to 250 mg, such as from 5 to 200 mg, for example from 5 to 150 mg, such as from 5 to 100 mg, for example from 5 to 75, such as from 5 to 50 mg dry buffer per ml liquid composition.

In embodiments of the invention wherein the liquid composition is a wine sample or a sample of partially fermented grape juice the predetermined amount of buffer is preferably in range of 2 to 450 mg, such as from 5 to 400 mg, for example from 5 to 350 mg, such as from 5 to 300 mg, for example from 5 to 250 mg, such as from 5 to 200 mg, for example from 5 to 150 mg, such as from 5 to 100 mg, for example from 5 to 75, such as from 5 to 50 mg, for example 5 to 25 mg dry buffer per ml wine or partially fermented grape juice.

In embodiments of the invention wherein the

buffer is $Na_2Citrate$ or $K_2HPO_4$, then preferably around 1 to 400 mg, for example from 2 to 350 mg, such as from 3 to 300 mg, for example from 4 to 250 mg, such as from 5 to 200 mg, for example from 5 to 150 mg, such as from 5 to 100 mg, for example from 5 to 75, such as from 5 to 50 mg, for example 5 to 25 mg, such as from 7 to 20, for example from 10 to 15 mg dry buffer per ml liquid composition is used.

In embodiments of the invention wherein the liquid composition is a wine sample or a sample of partially fermented grape juice and the buffer is $Na_2Citrate$ or $K_2HPO_4$, then preferably around 1 to 400 mg, such as from 3 to 300 mg, for example from 4 to 250 mg, such as from 5 to 200 mg, for example from 5 to 150 mg, such as from 5 to 100 mg, for example from 5 to 75, such as from 5 to 50 mg, for example 5 to 25 mg, such as from 7 to 20, for example from 10 to 15 mg, such as around 12 mg dry buffer per ml liquid composition is used.

Inhibitors

In some embodiments of the present invention it is preferred that an inhibitor of a metabolic pathway is added to the liquid composition. Alternatively, the composition comprising the microbial organism may comprise an inhibitor of a metabolic pathway (see herein below).

In other embodiments of the present invention it is preferred that a compound capable of specifically inhibiting yeast metabolism, but not bacterial metabolism is added to the liquid composition. Alternatively, the composition comprising the microbial organism may comprise a compound capable of specifically inhibiting yeast metabolism, but not bacterial metabolism (see herein below).

In particular, when the method comprises use of lactic acid bacteria to determine the amount of malic acid in a liquid composition, an inhibitor of a metabolic pathway and/or a compound capable of specifically inhibiting yeast metabolism, but not bacterial metabolism may be added.

Many liquid compositions comprise sugar, for example a wine sample or a sample of partially fermented grape juice may comprise residual sugar. A large number of microbial organisms are capable of fermenting sugar to produce i.a. $CO_2$. Furthermore, a wine sample or a sample of partially fermented grape juice may comprise yeast, which is capable of fermenting sugar.

Accordingly, if the amount of a fermentable compound, which is not sugar, is determined by correlation to $CO_2$ production, the result may be misleading due to the presence of $CO_2$ produced as a result of sugar fermentation. Analogous problems may be encountered due to the presence of other residual fermentable compounds in the liquid composition.

In order to circumvent this, the present invention provides use of inhibitors of selected metabolic pathways. The inhibitors may be a direct inhibitor of the selected metabolic pathway or it may only indirectly inhibit the metabolic pathway.

In embodiments wherein the fermentable compound is not a sugar, it is frequently desirable to inhibit sugar fermentation. This may be done by inhibiting the metabolic pathways of sugar fermentation or by specifically inhibiting microbial organisms capable of fermenting sugar.

Accordingly, the methods and compositions according to the present invention may furthermore comprise an inhibitor of a metabolic pathway. The metabolic pathway may for example be glycolysis.

An example of a inhibitor of glycolysis is NaF, however any other inhibitor of glycolysis may be used with the present invention.

Yeast as well as a number of bacteria are capable of fermenting sugar. However certain bacteria for example *Lactobacillus plantarum* do not produce $CO_2$ from sugar. Accordingly, in embodiments of the invention wherein a microbial organism, which cannot ferment sugar to produce $CO_2$ is used to determine the amount of a fermentable compound other than sugar, then the method may comprise adding a compound capable of specifically inhibiting yeast metabolism, but not bacterial metabolism.

An example of a compound capable of inhibiting yeast metabolism, but not bacterial metabolism is natamycin (available from Delvoid from Gist-brocades, Delft, Holland). However any other compound capable of specifically inhibiting yeast metabolism, but not bacterial metabolism may also be used with the present invention.

Determining the amount of a gaseous product

The amount of gaseous product produced during fermentation of the fermentable compound according to the present invention may be determined by any suitable method. The method should be selected according to the nature of the gaseous product.

In one embodiment the determination of the amount of gaseous product is based on measuring change in pressure. Because the volume of a gas is significantly larger than the volume of a liquid, production of a gas in a closed container will lead to a significant increase in pressure.

Accordingly, in one embodiment of the present invention determining the produced amount of assayable gaseous product involves the steps of

a) contacting said test microbial organism and said liquid composition in a confined space of an air tight container having a first pressure,
b) incubating said test microbial organism and said liquid composition for a predetermined period of time,
c) determining a second pressure in said confined space following said incubation, and

d) correlating said second pressure to the starting content of said at least one fermentable first compound present in said liquid composition, and thereby determining the produced amount of said at least one assayable gaseous product.

The first pressure will often be normal atmospheric pressure, i.e. from 0,5 til 1,5 atm, preferably around 1 atm.

Frequently, the liquid composition will be placed in a sealable container. The container may or may not be allowed to incubate with the liquid composition for example under shaking. Subsequently the microbial organism may be added and the container sealed in an airtight manner. Thus the first pressure will be equal to that of the surroundings, normally around 1 atm.

After incubation for a predetermined amount of time, the second pressure may be determined. The pressure may for example be determined by a manometer. Any conventional manometer known to the person skilled in the art may be used.

However, preferably, the manometer is mounted with means so it can be inserted into the airtight container. In one embodiment of the present invention the manometer has an injection needle mounted, which enables it to be inserted into an airtight container through a rubber sealing or the like. Accordingly, if the container is sealed with a rubber sealing or the like, the manometer preferably is mounted with an injection needle. Figure 1 depicts an example of an apparatus to be used with the present invention, which comprises a manometer mounted with a needle.

The apparatus for measuring the pressure may include a manometer as well as other components. In particular, determining the produced amount of assayable gaseous product may involve the use of an apparatus as described herein below.

Hence, in one preferred embodiment of the invention the pressure is determined by manometer comprising an injection needle and wherein said injection needle is injected into the container. In particular the second pressure may be determined by said manometer.

The predetermined amount of time for incubation of the liquid composition with the microbial organism should be selected according to the specific need. However, preferably, the predetermined amount of time is selected in order to allow complete fermentation of the fermentable compound to be assayed.

Accordingly, the predetermined amount of time should be selected according to the amount of fermentable compound present and the amount of microbial organism present. Because the amount of fermentable compound usually is unknown, the predetermined amount of time may be selected based on the amount of liquid composition and the amount of microbial organism.

Usually, the predetermined period of time is in the range of 1 min to 24 hours, such as from 2 min to 12 hours, for example from 5 min to 8 hours, such as from 5 min to 5 hours, such as from 10 min to 4 hours, for example from 30 min to 3 hours, such as from 30 min to 2 hours.

In one embodiment of the present invention the predetermined amount of time is preferably around 60 min. In particular, when around 40 mg dried yeast, such as *Saccharomyces cerevisiae* per ml liquid composition is used, the predetermined amount of time is preferably in the range of 5 min to 5 hours, more preferably, from 10 min to 4 hours, even more preferably from 20 min to 3 hours, yet more preferably from 30 min to 2 hours, most preferably around 1 hour.

Furthermore, when around 80 mg dried bacteria, such as *Oenococcus oeni* or *Lactobacillus plantarum* per ml liquid composition is used the predetermined amount of time is preferably in the range of 5 min to 5 hours, more preferably, from 10 min to 4 hours, even more preferably from 20 min to 3 hours, yet more preferably from 30 min to 2 hours, most preferably around 1 hour.

Test sample container

The test sample container should be selected so that it is suitable for the methods used for determining the amount of gaseous product (see herein above). When the amount of gaseous product is determined by use of manometric methods, then the container is preferably an airtight container.

In embodiments wherein the amount of gaseous product is determined by use of manometer mounted with an injection needle, the container is preferably air tight, pressure stable and penetrable by a needle. In order to be penetrable by a needle, the container may for example comprise a part, such as a lid, which is manufactured of rubber or the like. In one preferred embodiment the container may be sealed with a crimp cap with septa.

The container may be prepared of any suitable material, for example glass, plastic or metal, preferably however, the container is prepared of glass.

In one preferred embodiment of the invention the test sample container is a glass vial closed with a penetratable sealing cap.

Predetermined fermentation parameter

The present invention provides methods of monitoring a fermentation process involving defining a predetermined fermentation parameter and subsequently comparing the amount of fermentable compound to the predetermined fermentation parameter.

The predetermined fermentation parameter should be selected according to the fermentable compound and the desired outcome of the fermentation process. For example it may for some purposes be desirable that essentially no fermentable compound is left in the liquid composition after the fermentation process, and for other purposes it may be desirable that there is a specific amount of fermentable compound left in the liquid composition after the fermentation.

In wine production for example it may be desirable to produce a dry wine with essentially no residual sugar or it may be desirable to produce a sweet wine which contains residual sugar.

In one embodiment of the present invention the predetermined parameter is in the range of 0 to 100 g/l, such as 0 to 80 g/l, for example 0 to 60 g/l, such as 0 to 40 g/l, for example 0 to 20 g/l, such as 0 to 15 g/l, for example 0 to 10 g/l, such as 0 to 8 g/l, for example 0 to 6 g/l, such as 0 to 4 g/l, for example 0 to 3 g/l, such as 0 to 2 g/l, for example 0 to 1 g/l, such as 0 to 0.75 g/l, for example 0 to 0.5 g/l, such as 0 to 0.4 g/l, for example 0 to 0.3 g/l, such as 0 to 0.2 g/l, for example 0 to 0.1 g/l, such as around 0 g/l.

In particular, when the desired outcome of the fermentation process is a dry wine the predetermined parameter may be as mentioned above. Furthermore, if the fermentable compound is selected from the group consisting of glucose, fructose, sucrose and malic acid the predetermined parameter may be as mentioned above.

Once the predetermined fermentation parameter has been defined for the particular embodiment of the invention, the amount of fermentable compound may be determined as described herein. The amount of fermentable compound may then be compared with the predetermined fermentation parameter.

According to said comparison the fermentation process may be controlled. For example, the fermentation process may be allowed to proceed, if the amount of fermentable compound is higher than the predetermined fermentation parameter.

After a timely interval the method may be repeated and the newly determined amount of fermentable compound may be compared with the predetermined fermentation parameter. Again the fermentation process may be allowed to proceed, if the amount of fermentable compound is higher than the predetermined fermentation parameter.

When the amount of fermentable compound is equal to or lower than the predetermined fermentation parameter, then for example the fermentation process may be terminated or the next step in the fermentation process may be initiated. The fermentation process may be terminated by any conventional means, for example by treatment with sulphur. The subsequent fermentation step may be initiated for example by addition of a microbial organism different to the microbial organism used in the first fermentation step.

Predetermined time of incubation

The present invention provides methods for controlling a fermentation process, wherein a liquid composition is incubated with a microbial organism for a predetermined time of incubation, after which a test sample is acquired from said liquid composition and the amount of at least one first fermentable compound in said test sample is determined.

The predetermined time of incubation should be selected according to the liquid composition, the desired end product of the fermentation process and the fermentable compound to be determined.

In general the predetermined amount of time is in the range of 1 to 2 days, such as 2 to 4 days, for example 4 to 7 days, such as 1 to 2 weeks, for example 2 to 5 weeks, such as 1 to 2 months, for example 2 to 3 months, such as 3 to 5 months, for example 5 to 12 months, such as 1 to 2 years, for example more than 1 year. In embodiments wherein the fermentation process is wine production, it is often desirable to monitor the fermentation of sugar and the malolactic fermentation. Usually, the predetermined amount of time for measuring the amount of sugar is shorter than the predetermined amount of time for measuring malic acid.

Preferably, the amount of malic acid is determined approximately 0 to 50 weeks, such as 0 to 25 weeks, for example 0 to 20 weeks, such as 0 to 16 weeks, for example 0 to 1 weeks after the fermentation of sugar has finished. In general the predetermined time of incubation when the amount of malic acid should be determined is in the range of 0 to 50 weeks, such as in the range of 1 to 30 weeks, for example in the range of 1 to 16 weeks.

Methods

The present invention describes methods for determining the amount of a fermentable compound in a liquid composition.

The methods involve contacting the liquid composition with a microbial organism capable of fermenting said fermentable compound and thereby producing an assayable gaseous product. The liquid composition and the microbial organism should be incubated under conditions allowing the microbial organism to ferment essentially all of the fermentable compound. Subsequently, the amount of gaseous product may be determined and correlated to the amount of fermentable compound.

Contacting the liquid composition with microbial organism may be done by any convenient method known to the skilled person.

In one embodiment of the present invention one of the dry compositions comprising a microbial organism described herein below is added directly to the liquid composition. For example in the range of 0.05 to 5 g, such as in the range of 0.1 to 3 g, for example 0.2 to 1 g, such as in the range from 0.3 to 0.7 g dry composition may be added per 5 ml. liquid composition.

In another embodiment of the present invention the microbial organism may be resuspended in a liquid medium, such as an aqueous suspension, prior to contacting the liquid composition. For example any of the dry compositions mentioned herein below may be resuspended in a liquid medium and the suspension is added to the liquid composition. The liquid medium may be any suitable liquid medium, however preferably the liquid medium is water, such as tap water, distilled water or sterile water.

The dry composition may be resuspended in the liquid medium in any useful relationship. For example, in the range of 1 to 50 g, such as 3 to 40 g, for example 5 to 30 g, such as 6 to 25 g, for example 7 to 20 g, such as 8 to 15 g, for example 9 to 14 g, such as 10 to 13 g dry composition may be added to 40 ml liquid medium, such as water. Preferably, the suspension may be stored at 4°C for at least 1 day, such as 2 days, for example 3 days, such as a week, for example 2 weeks and still be employed with the methods according to the invention.

In one example wherein the microbial organism is a bacteria, around 12.5 g dry composition may be added to around 40 ml water. In another example wherein the microbial organism is yeast around 11 g dry composition may be added to around 40 ml water. From said suspensions in range of 100 µl to 10 ml, such as 0.5 to 7 ml, for example 1 to 5 ml, such as 1,5 to 3 ml, for example around 2 ml may be added per 5 ml of liquid composition. The term "essentially all of the fermentable compound" within the context of the present invention means all or nearly all of the fermentable product, for example at least 80%, such as at least 85%, for example at least 90%, such as at least 95%, for example at least 98%, such as at least 99%, for example at least 99.5%, such as at least 99.9%, for example around 100% of the fermentable compound. Preferably, "essentially all of the fermentable compound" means all detectable fermentable compound.

The conditions allowing the microbial organism to ferment essentially all of the fermentable compound should be selected according to the liquid composition and the nature of the microbial organism.

Conditions may include incubation for a predetermined period of time. Preferred predetermined period of time is discussed herein above. Furthermore, condition may include incubation at a specific temperature. For most purposes the temperature is room temperature i.e. a temperature in the range of 10°C to 30°C, such as in the range from 15°C to 25°C.

In addition conditions may comprise shaking, stirring or the like. Shaking may be done using any conventional means therefore, for example the liquid composition and the microbial organism mixture may be incubated in a container fixed to a shaking table.

The mixture may be submitted to shaking during the entire incubation period or the shaking may be applied only during certain intervals of the incubation period. For example the mixture may be submitted to shaking for 1 to 2 min., such as from 2 to 5 min, for example from 5 to 10 min, such as from 10 to 20 min., for example from 20 to 30 min, such as from 30 to 45 min, for example from 45 to 60 min., such as for more than 60 min. The mixture may be submitted to shaking once, such as twice, for example 3 to 5 times, such as 5 to 10 times, for example 10 to 20 times during incubation.

Subsequently, the amount of gaseous product produced may be determined by any available method, for example any of the methods described herein above.

Once the amount of gaseous product is determined either directly or indirectly, this may be correlated to the amount of fermentable compound that was present in the liquid composition. Correlation may for example be done by the aid of a standard curve.

If the amount of gaseous product is determined by determining change in pressure, said change in pressure may directly be correlated to the amount of fermentable compound.

In one embodiment of the present invention the first pressure is the normal pressure of the surroundings, i.e. around 1 atm and hence relatively constant. The second pressure may then be directly correlated to the amount of fermentable compound. Preferably, correlation may be done using a standard curve.

Preferably, the second pressure is linearly correlatable with the amount of fermentable compound over the range of interest. The range of interest depends on the fermentable compound, but is for example 0 to 200 g, such as 0 to 100 g, for example 0 to 75 g, such as 0 to 50 g, such as 0 to 25 g, such as 0 to 14 g, for example 0 to 10 g, such as 0 to 7 g fermentable compound per litre liquid composition.

The standard curve is preferably prepared by determining the second pressure using various known predetermined amounts of fermentable compound within the range of interest. It is preferred that the second pressure is determined for at least 2, such as at least 3, for example at least 4, such as at least 5, such as at least 6, for example at least 7, such as at least 8 different known predetermined amounts of fermentable compound. Hence, the second pressure may be determined for in the range of 2 to 50, such as 3 to 40, for example 4 to 30, such as 5 to 25, for example 6 to 20, such as 7 to 15 different known predetermined amounts of fermentable compound.

Furthermore preferably, the second pressure is independently determined at least once, such as twice, for example 3 times, such as 4 times, for example 5 times, such as more than 5 times for each known predetermined amount of fermentable compound.

Once the second pressures have been determined a standard curve may be prepared. Preferably, the standard curve is prepared using linear regression, wherein the second pressure may be indicated as a function of the amount of fermentable compound. When the standard curve is prepared using linear regression it is preferred that R-square ($R^2$) is larger than 0.8, such as larger than 0.85, for example larger than 0.9, such as larger than 0.95, for example larger than 0.96, such as larger than 0.97, for example larger than 0.98, such as larger than 0.99, for example in the range of 0.99 to 1.

Figure 2 and figure 3 depicts examples of standard curves that may be used with the present invention, wherein the second pressure is directly correlatable with the amount of either glucose, fructose or malic acid in a wine sample. Said standard curves may be used when the methods are performed as described in example 1 or 2.

If the methods according to the invention are performed differently from example 1 or 2, standard curves adapted for the particular methods may be made, by performing the methods using various liquid compositions with known contents of the fermentable compound.

In one particular embodiment of the present invention the second pressure is determined by use of a manometer, which has been calibrated to directly measure the amount of fermentable compound. Accordingly, in such an embodiment of the invention the amount of fermentable compound, may be directly displayed on the manometer.

The method of determining the amount of a fermentable compound in a liquid composition may be repeated as often as desirable. In one embodiment of the present invention the methods are used to monitor a fermentation process. For example the method may comprise the steps outlined herein above.

The methods may be performed regularly at timely intervals during a fermentation process to determine when the desired amount of fermentable compound is present in the liquid composition, i.e. when the liquid composition comprises a predetermined fermentation parameter (see herein above).

Accordingly, during a fermentation process samples may be withdrawn at timely intervals and the methods may be performed on said samples. For example the methods may be performed once, such as twice, for example 3 to 5 times, such as 5 to 10 times, for example 10 to 20 times, such as 20 to 30 times, for example 30 to 50 times, such as 50 to 100 times, such as more than 100 times.

The samples may be withdrawn at regular timely intervals or they may be withdrawn with varying timely intervals. For example the timely intervals may individually be in the range of 1 to 60 min, such as 1 to 24 hours, for example 1 to 5 days, such as 5 to 10 days, for example 10 to 20 days, such as 20 to 50 days, such as 50 to 100 days, for example more than 100 days.

The methods may be repeated to measure the amount of one fermentable compound or they may be repeated to measure the amount of different fermentable compounds.

Hence, during a fermentation process samples may be withdrawn to determine the amount of a first fermentable compound according to the methods described herein; and/or samples may be withdrawn to determine the amount of a second fermentable compound; and/or samples may be withdrawn to determine the amount of a third fermentable compound; and/or samples may be withdrawn to determine the amount of a fourth fermentable compound; and/or samples may be withdrawn to determine the amount of a fifth fermentable compound; and/or samples may be withdrawn to determine the amount of further fermentable compounds.

In embodiments of the present invention wherein fermentation of grape juice to produce wine is monitored, then it is frequently desirable to monitor the amount of residual sugar (for example glucose, sucrose and/or fructose) in the grape juice/wine during the first part of the fermentation process and subsequently monitor the amount of malic acid during the second part of the fermentation process.

In preferred embodiments of the present invention the methods as described herein involves the use of any of the compositions described herein below comprising a microbial organism.

Apparatus

The present invention also relates to an apparatus, which may be used in the methods described herein above. Accordingly, in one aspect the invention relates to an apparatus for determining the amount of at least one fermentable first compound in a liquid composition, wherein said apparatus comprises

> a) a manometer comprising an injection needle for measuring the pressure within an air tight, test sample container comprising a confined space, wherein said manometer is calibrated to measure the amount of said at least one fermentable first compound; and
> b) an air tight, test sample container, wherein said container is pressure stable and penetrable by said injection needle; and
> c) a display capable of directly displaying the amount of said at least one fermentable first compound.

The manometer per se may be any conventional manometer upon which an injection needle can be mounted. The injection needle should be mounted in a manner so that pressure is measured through the injection needle. Accordingly, the injection needle may be stuck into an airtight container and thereby the pressure inside the airtight container may be measured.

The manometer should preferably be calibrated to measure the amount of at least one fermentable compound and comprise a display, which directly displays the amount of said at least one fermentable compound. The manometer may be calibrated by aid of a standard curve as described herein above.

It is also contained within the present invention that the manometer is calibrated to measure more than one different fermentable compounds. Furthermore, the manometer may comprise a display capable of displaying the amount of more than one fermentable compound.

The display may be any conventional display capable of displaying numeric values, for example any analogue or digital display. Accordingly, the apparatus may further comprise an output unit displaying the amount of said at least one fermentable first compound.

The air tight, test sample container may be any of the test sample containers described herein above. Preferably the container is pressure stable and penetrable by an injection needle, more preferably, the container is a glass vial closed with a penetrable sealing cap.

The apparatus may apart from the above mentioned parts comprise further parts. In one embodiment the apparatus furthermore comprises a shaker. The shaker may be any conventional shaker known to the person skilled in the art, for example a shaking table. The shaker may or may not comprise a timer, that controls period of shaking.

The apparatus may comprise a movable sledge, i.e. a sledge which can move back and forward. Preferably it is possible to place a solid support capable of fixation of at least one test sample container upon said sledge. Accordingly, it is possible to shake a test sample container on said sledge, by moving it back and forward. The sledge may be moved by a motor, for example an electromotor. Said motor may or may not be controlled by a timer.

In another embodiment the apparatus comprises a solid support capable of fixation of at least one test sample container. Any solid support capable of fixation of at least one test sample container may be used with the present invention, such as for example a rack. Accordingly, the solid support should be selected according to the shape of the test sample container.

The solid support may be capable of fixating more than one test sample container, such as from 1 to 1000, for example from 1 to 500, such as from 1 to 100, for example from 1 to 75, such as from 1 to 50, for example from 2 to 40, for example from 5 to 30, such as from 10 to 25, for example from 15 to 25, such as around 20.

In one embodiment of the present invention the solid support is mounted on the shaker.

Examples of apparatuses according to the present invention are depicted in figure 1 and figure 4, respectively.

Composition

In one aspect the present invention relates to compositions comprising a dried microbial organism capable of fermenting said fermentable compound; and

a) a buffer compound capable of buffering a solution to a pH in the range of from 4,0 to 8,0; or
b) a solid inhibitor of at least one metabolic pathway producing a gaseous product; or
c) a solid compound capable of inhibiting yeast metabolism, but not bacterial metabolism.

Said compositions are preferably dry compositions, which allows long term storage of the compositions without major loss of viability of the microbial organisms.

The dry compositions are preferably stored at a temperature in the range of -80°C to 40°C, such as from -40 °C to 40 °C, for example from -25°C to -10°C, such as from -10°C to 0°C, for example from 0°C to 40°C, such as from 0°C to 30°C, for example 0°C to 20°C, such as 0°C to 10°C. More preferably, the dry compositions may be stored at a temperature at for example around 4°C, such as around -20°C or for example around -40°C.

The buffer may be any of the buffers as described herein above, preferably a buffer capable of buffering a solution, such as grape juice, partially fermented grape juice or wine to a pH in the range of from 4,0 to 8,0.

The inhibitor of at least one metabolic pathway may be any inhibitor described herein above, preferably the inhibitor is a solid inhibitor of at least one metabolic pathway producing a gaseous product. For example the inhibitor may be NaF.

The compound capable of specifically inhibiting yeast metabolism, but not bacterial metabolism, may be any of the compounds described herein above. Preferably, the compound is provided as a solid compound, for example natamycin.

In one embodiment of the present invention the composition comprises a microbial organism and a dry buffer. Preferably, the composition comprises in range from 1 g to 2000 g, such as 10 g to 1000 g, for example 20 g to 1500 g, such as 30 g to 1000 g, for example 50 g to 800 g, such as 75 g to 700 g, for example 100 g to 600 g, such as 150 g to 500 g, for example 200 g to 400 g, such as 250 g to 350 g dry bufffer per 1000 g dry microbial organism.

In embodimentes of the invention wherein the microbial organism is dried *Saccharomyces cerevisiae* and the buffer compound is $Na_2Citrate$ or $K_2HPO_4$, the composition preferably comprises 10 g to 1000 g, for example 20 g to 1500 g, such as 30 g to 1200 g, for example 50 g to 1000 g, such as 75 g to 900 g, for example 100 g to 800 g, such as 150 g to 700 g, for example 200 g to 600 g, such as 300 g to 600 g, for example 400 g to 600 g, such as around 500 g $Na_2Citrate$ or $K_2HPO_4$ per 1000 g dried *Saccharomyces cerevisiae.* Preferably, the composition comprises around 500 g $Na_2Citrate$ or $K_2HPO_4$ pr. 1000 g yeast.

The compositions according to the present invention may furthermore comprise one or more antibiotics. In particular, in embodiments of the invention, wherein the microbial organism is yeast, the compositions may comprise one or more antibiotics. The compositions according to the invention may be placed under conditions wherein microbial growth is possible or even promoted, and it may therefore be advantageous to inhibit bacterial growth. For example, the composition may be resuspended in an aqueous medium. In order to prolong the stability of a composition comprising yeast in an aqueous environment bacterial growth is preferably inhibited.

Any antibiotic available to the person skilled in the art may be used with the invention, for example penicillin, bacitracin, chloramphenicol or valinomycin. In one preferred embodiment, the antibiotic is chloramphenicol.

The antibiotic should be added to the compositions in amounts sufficient to inhibit bacterial growth. Hence, preferably the composition comprises in the range from 0.01 g to 100 g, such as 0.05 g to 50 g, for example 0.1 g to 10 g, such as 0.2 g to 5 g, for example 0.3 g to 2 g, such as 0.4 g to 1 g, for example 0.5 g to 0,8 g, such as 0.6 g to 0,7 g dry antibiotic per 1000 g dry microbial organism.

In embodiments of the invention wherein the microbial organism is dried *Saccharomyces cerevisiae* and the antibiotic is chloramphenicol, then the composition may comprise in the range from 0.01 g to 100 g, such as 0.05 g to 50 g, for example 0.1 g to 10 g, such as 0.2 g to 5 g, for example 0.3 g to 2 g, such as 0.4 g to 1 g, for example 0.5 g to 0,8 g, such as 0.6 g to 0,7 g dry chloramphenicol per 1000 g dry *Saccharomyces cerevisiae.* Preferably, the composition comprises around 0.68 g dry chloramphenicol per 1000 g dry *Saccharomyces cerevisiae.*

In some embodiments of the present invention the compostions are intended for resuspension in an aqueous medium prior to contacting a liquid comprising a fermentable compound with said composition. It is then preferred that the aqueous medium wherein the composition is resuspended comprises in the range of 0.001 % to 1%, more preferably in the range from 0.002% to 0.5%, even more preferably in the range from 0,005% to 0.1%, yet more preferably in the range from 0.007% to 0.05%, even more preferably around 0.01% antibiotic, for example chloramphenicol.

In another embodiment of the present invention the composition comprises a microbial organism and an inhibitor of a metabolic pathway. Preferably, the composition comprises in range from 0.1 g to 1000 g, such as 0.2 g to 500 g, for example 0.3 g to 300 g, such as 0.4 g to 200 g, for example 0.5 g to 100 g, such as 0.6 g to 50 g, for example 0.7 g to 25 g, such as 0.8 g to 20 g, for example 0.9 g to 15 g, such as 1 g to 10 g dry inhibitor per 1000 g dry microbial organism.

In embodimentes of the invention wherein the microbial organism is freeze dried bacteria and the inhibitor is NaF, the composition preferably comprises in range from 0.1 g to 200 g, for example 0.5 g to 100 g, such as 0.6 g to 50 g, for example 0.7 g to 25 g, such as 0.8 g to 20 g, for example 0.9 g to 15 g, such as 1 g to 10 g, for example 1.5 g to 5 g, such as 2 g to 4 g, for example 2 g to 3 g, such as around 2.5 g dry NaF per 1000 g dry bacteria. Preferably, the composition comprises around 2,5 g NaF pr. 1000 g bacteria.

**Examples**

The following examples illustrates selected embodiments of the invention and should not be considered as limiting for the invention.

Example 1

Determination of residual sugar in a wine sample

The method involves the use of an apparatus as depicted in figure 4.

A composition consisting of 500 g Na$_2$Citrate and 1000 g dried yeast (either "Lalvin V1116" produced by Lallemand, Montreal, Canada or "Malteser Tørgær" from "De Danske Spritfabrikker", Grenaa, Denmark) is prepared.

First two standard curves are prepared. 0 g/l, 2 g/l, 4 g/l, 6 g/l, 8 g/l. 10 g/l, 12 g/l or 14 g/l glucose or fructose is added to a 5 ml completely fermented wine sample. The wine sample is red wine produced from Biotta grape juice, which has completed alcohol fermentation. Wine data: 12% ethanol (v/v), pH 3.5, 0 g glucose/L, 0 g fructose/L.

The measurement is repeated 4 times for each glucose concentration and 4 times for each fructose concentration. The values indicated below are average values for the 4 individual determinations.

The samples are added to 20 ml glass bottles. The bottles are shaken for 2 minutes in order to remove $CO_2$ within the sample. 0.6 g of the yeast composition is added to the bottles and the bottles are shaken for additional 2 minutes. Subsequently, the bottles are closed with airtight crimp caps with septa and incubated at room temperature for 60 minutes. The bottles are shaken for 4 minutes and the pressure within the bottles is determined by sticking the injection needle of the manometer into the bottles and measuring the pressure.

Individual standard curves are prepared for pressure as a function of glucose content or fructose content (figure 2).

| Glucose (g/L) | Pressure (PSI) |
|:---:|:---:|
| 0 | 0 |
| 2 | 3,38 |
| 4 | 6,625 |
| 6 | 9,68 |
| 8 | 12,75 |
| 10 | 15,55 |
| 12 | 18,53 |
| 14 | 21,08 |

Standard curve for glucose:

$$y = 1,5069x + 0,4012$$

$$R^2 = 0,9986,$$

wherein y is the pressure (PSI) and x is glucose content (g/L).

| Fructose (g/L) | Pressure (PSI) |
|---|---|
| 0 | 0 |
| 2 | 3,05 |
| 4 | 6,325 |
| 6 | 9,425 |
| 8 | 12,542 |
| 10 | 15,425 |
| 12 | 18,525 |
| 14 | 21,25 |

Standard curve for fructose:

$$y = 1,527x + 0,1285$$

$$R2 = 0,9996$$

wherein y is the pressure (PSI) and x is fructose content (g/L).

The manometer is calibrated so display the amount of either glucose or fructose in a sample using the standard curves.

The glucose or fructose content of wine samples are then determined. 5 ml wine samples are added to 20 ml glass bottles (one sample per bottle). The bottles are shaken for 2 minutes in order to remove $CO_2$ within the sample. 0.6 g of the yeast composition is added to the bottles and the bottles are shaken for additional 2 minutes. Subsequently, the bottles are closed with airtight crimp caps with septa and incubated at room temperature for 60 minutes. The bottles are shaken for 4 minutes and the amount or glucose or fructose within the bottles is determined by sticking the injection needle of the manometer into the bottles and reading the display.

Example 2

Determination of malic acid in a wine sample
The method involves the use of an apparatus as depicted in figure 4.
A composition consisting of 2.5 g NaF and 1000 g dried bacteria (either Viniflora oenos or Viniflora LP, both available from Chr. Hansen, Hørsholm, Denmark) is prepared.

First a standard curve is prepared. 0 g/l, 1 g/l, 2 g/l, 3 g/l, 4 g/l, 5 g/l, 6 g/l or 7 g/l malic acid is added to a 5 ml wine sample devoid of malic acid (all malic acid has previously been fermented to lactic acid). The wine sample is red wine produced from Biotta grape juice, which has completed malolactic fermentation. Wine data: 12% ethanol (v/v), pH 3.5, 0 g malic acid/L.
The measurement is repeated 4 times for each malic acid concentration. The values indicated below are average values for the 4 individual determinations.
The samples are added to 20 ml glass bottles. The bottles are shaken for 2 minutes in order to remove $CO_2$ within the sample. 0.4 g of the bacteria composition is added to the bottles and the bottles are shaken for additional 2 minutes. Subsequently, the bottles are closed with airtight crimp caps with septa and incubated at room temperature for 60 minutes. The bottles are shaken for 4 minutes and the pressure within the bottles is determined by sticking the injection needle of the manometer into the bottles and measuring the pressure.
A standard curve is prepared for pressure as a function of malic acid content (figure 3).

| Malic acid (g/L) | Pressure (PSI) |
|---|---|
| 0 | 0 |
| 1 | 1,2 |
| 2 | 2,325 |
| 3 | 3,45 |
| 4 | 4,525 |
| 5 | 5,675 |
| 6 | 6,825 |
| 7 | 7,933 |

Standard curve for malic acid:

$$y = 1,1283x + 0,0424,$$

$$R^2 = 0,9999,$$

wherein y is pressure (PSI) and x is malic acid content (g/L)
The manometer is calibrated to display the amount of malic acid in a sample using the standard curve.
The malic acid content of wine samples is then determined. 5 ml wine samples are added to 20 ml glass bottles (one sample per bottle). The bottles are shaken for 2 minutes in order to remove $CO_2$ within the sample. 0.4 g of the yeast composition is added to the bottles and the bottles are shaken for additional 2 minutes. Subsequently, the bottles are closed with airtight crimp caps with septa and incubated at room temperature for 60 minutes. The bottles are shaken for 4 minutes and the amount of malic acid within the bottles is determined by sticking the injection needle of the manometer into the bottles and reading the display.

**Example 3**

A composition consisting of 500 g $Na_2$Citrate and 1000 g dried yeast (either "Lalvin V1116" produced by Lallemand, Montréal, Canada or "Malteser Tørgær" from "De Danske Spritfabrikker", Grenaa, Denmark) is prepared.

To 11 g of said composition 5 mg chloramphenicol (Fluka Chemie GmbH, Industriestrasse 25, CH-9471 Buchs, Switzerland.) is added and the composition is then resuspended in 40 ml. $H_2O$.

The method described in example 1 may then be performed, wherein 2 ml of the suspension is added to the glass bottles instead to 0.6 g dry yeast composition. References

Bach HP, Fay JP and Baltes-Gotz B, Wein-Wiss., 1992, A reproducible measurement of carbon dioxide desorption from sparkling wines, 47: 46-52

Miranda M, Ramos A, Veiga-Da-Cuncha M, Loureiro-Dias MC and Santos H. J Bacteriology, 1997, Biochemical basis for glucose-induced inhibition of malolactic fermentation in *Leuconostoc oenos*, 179: 5347-5354.

Rammert M and Pahl MH, Brauwelt, 1992, Solubility of carbon dioxide in beverages, 132:490-9

Williams S, Official Methods of Analysis 14th edition, 1984, Association of official Analytical Chemists, p. 226-227.

**Claims**

1. A method for determining the amount of malic acid in a fruit juice or a fermented fruit juice, said method comprising the steps of

    i) providing a fruit juice or a fermented fruit juice,
    ii) providing a test microbial organism capable of fermenting malic acid to lactic acid and thereby producing $CO_2$,
    iii) contacting said fruit juice or fermented fruit juice with said test microbial organism,
    iv) adding an inhibitor of glycolysis,
    v) fermenting essentially all malic acid, wherein said fermentation results in the production of $CO_2$ in an amount directly correlatable with the amount of malic acid present in the fruit juice or fermented fruit juice,
    vi) determining the produced amount of $CO_2$,
    vii) correlating the produced amount of $CO_2$ with the amount of malic acid, and thereby determining the amount of malic acid.

2. The method of claim 1, wherein the step of determining the produced amount of $CO_2$ involves

    a) contacting said test microbial organism and said fruit juice or fermented fruit juice in a confined space of an air tight container having a first pressure,
    b) incubating said test microbial organism and the fruit juice or fermented fruit juice for a predetermined period of time,
    c) determining a second pressure in said confined space following said incubation, and
    d) correlating said second pressure to the starting content of malic acid present in the fruit juice or fermented fruit juice, and thereby determining the produced amount of $CO_2$.

3. The method of claim 1, wherein the method is employed for controlling a fermentation process, said fermentation process comprising the steps of

    i) providing a fruit juice or a fermented fruit juice,
    ii) providing a microbial organism capable of fermenting malic acid,
    iii) defining a predetermined fermentation parameter,
    iv) incubating said fruit juice or fermented fruit juice with said microbial organism under conditions allowing said microbial organism to ferment at least part of the malic acid,
    v) obtaining from said liquid composition, after a predetermined time of incubation, a test sample,
    vi) determining the amount of malic acid according to the method of any of claims 1 and 2; and
    vii) comparing the amount of malic acid with said predetermined fermentation parameter, and
    viii) controlling said fermentation process based on the comparison of the amount of said malic acid with said predetermined fermentation parameter.

4. The method of any of claims 1 to 3, wherein the fruit juice or fermented fruit juice is grape juice or fermented grape juice.

5. The method according to any of claims 1 to 3, wherein the inhibitor is NaF.

6. The method of any of claims 1 to 3, wherein the microbial organism is selected from the group of bacteria belonging to the *Oenococcus* family and the *Lactobacillus* family.

7. The method of any claims 1 to 3, wherein the test microbial organism is in dried form.

8. The method of any of claims 2 to 3, wherein the container is air tight, pressure stable and penetrable by a needle.

9. The method according to any of claims 2 to 3, wherein the pressure is determined by manometer comprising an injection needle and wherein said injection needle is injected into the container.

10. The method according to claim 2, wherein said predetermined period of time is in the range of 5 min to 5 hours.

11. The method according to claim 1, wherein the test microbial organism is resuspended in a liquid medium prior to contacting said liquid composition.

12. A solid composition for determining the amount of malic acid in a fruit juice or fermented fruit juice, said solid composition comprising a dried microbial organism capable of fermenting malic acid to lactic acid thereby producing $CO_2$ and a solid inhibitor of glycolysis

13. The composition according to claim 12, wherein the bacteria is selected from the group of bacteria belonging to the *Oenococcus* family and the *Lactobacillus* family.

14. The composition according to claim 12, wherein the inhibitor is NaF.

15. The composition according to claim 12, wherein the microbial organism is freeze dried bacteria and the inhibitor is NaF.

16. The composition according to claim 15, wherein the composition comprises around 2,5 g NaF pr. 1000 g bacteria.

17. The method according to any of claims 1-11, wherein the method involves the use of a composition according to any of claims 12 to 16.

**Patentansprüche**

1. Verfahren zur Bestimmung der Menge an Maleinsäure in einem Fruchtsaft oder einem fermentierten Fruchtsaft, wobei das Verfahren die Schritte umfasst:

    i) Bereitstellen eines Fruchtsaftes oder eines fermentierten Fruchtsaftes,
    ii) Bereitstellen eines Test-Mikroorganismusses, welcher Maleinsäure zu Milchsäure fermentieren kann und dabei $CO_2$ produziert,
    iii) Inkontaktbringen des Fruchtsaftes oder des fermentierten Fruchtsaftes mit dem Test - Mikroorganismus,
    iv) Zugeben eines Inhibitors der Glykolyse,
    v) Fermentieren von im wesentlichen der gesamten Maleinsäure, wobei die Fermentation zur Herstellung von $CO_2$ in einer Menge führt, welche direkt mit der Menge von in dem Fruchtsaft oder dem fermentierten Fruchtsaft vorhandenen Maleinsäure korreliert werden kann,
    vi) Bestimmen der hergestellten Menge an $CO_2$,
    vii) Korrelieren der hergestellten Menge an $CO_2$ mit der Menge an Maleinsäure, und **dadurch** Bestimmen der Menge an Maleinsäure.

2. Verfahren nach Anspruch 1, wobei der Schritt der Bestimmung der hergestellten Menge an $CO_2$ beinhaltet,

    a) Inkontaktbringen des Test-Mikroorganismusses und des Fruchtsaftes oder des fermentierten Fruchtsaftes in einem begrenzten Raum eines Luftdichten Behälters mit einem ersten Druck,
    b) Inkubieren des Test- Mikroorganismus und des Fruchtsaftes oder des fermentierten Fruchtsaftes für eine bestimmte Zeitspanne,
    c) Bestimmen eines zweiten Druckes in dem begrenzten Raum nach der Inkubation, und
    d) Korrelieren des zweiten Drucks mit dem in dem Fruchtsaft oder in dem fermentierten Fruchtsaft vorhanden Ausgangsgehalt an Maleinsäure, und **dadurch** Bestimmen der hergestellten Menge an $CO_2$.

3. Verfahren nach Anspruch 1, wobei das Verfahren zur Steuerung eines Fermentations-prozesses eingesetzt wird, wobei der Fermentationsprozess die Schritte umfaßt:

    i) Bereitstellen eines Fruchtsaftes oder eines fermentierten Fruchtsaftes,
    ii) Bereitstellen eines Mikroorganismusses, welche Maleinsäure fermentieren kann,
    iii) Definieren eines bestimmen Fermentation - Parameters,
    iv) Inkubieren des Fruchtsaftes oder fermentierten Fruchtsaftes mit dem Mikroorganismus unter Bedingungen, bei denen der Mikroorganismus mindestens einen Teil der Maleinsäure fermentieren kann,
    v) Erhalten einer Testprobe aus der flüssigen Zusammensetzung nach einer bestimmten Zeitspanne der Inkubation,
    vi) Bestimmen der Menge an Maleinsäure gemäß dem Verfahren nach einen der Ansprüche 1 und 2, und
    vii) Vergleichen der Menge an Maleinsäure mit dem bestimmen Fermentations-Parameter, und

viii) Steuern des Fermentations-Prozesses basierend auf dem Vergleich der Menge an Maleinsäure mit dem bestimmten Fermentations-Parameter.

4. Verfahren nach einem der Ansprüche 1-3, wobei der Fruchtsaft oder der fermentierte Fruchtsaft Traubensaft oder fermentierter Traubensaft ist.

5. Verfahren nach einem der Ansprüche 1-3, wobei der Inhibitor NaF ist.

6. Verfahren nach einem der Ansprüche 1-3, wobei der Mikroorganismus ausgewählt ist aus der Gruppe von Bakterien, welche zu der *Oenococcus*-Familie und der *Lactobacillus*-Familie gehört.

7. Verfahren nach einem der Ansprüche 1-3, wobei der Test-Mikroorganismus in trockener Form vorliegt.

8. Verfahren nach einem der Ansprüche 2-3, wobei der Behälter luftdicht, druckstabil und mit einer Nadel durchdringbar ist.

9. Verfahren nach einem der Ansprüche 2-3, wobei der Druck durch ein Manometer bestimmt wird, welches eine Injektionsnadel umfaßt, und wobei die Injektionsnadel in den Behälter eingebracht wird.

10. Verfahren nach Anspruch 2, wobei die bestimmte Zeitspanne im Bereich von 5 Min. bis 5 Std. liegt.

11. Verfahren nach Anspruch 1, wobei der Test- Mikroorganismus vor dem Inkontaktbringen mit der flüssigen Zusammensetzung in einem flüssigen Medium resuspendiert wird.

12. Feste Zusammensetzung zur Bestimmung der Menge an Maleinsäure in einem Fruchtsaft oder fermentierten Fruchtsaft, worin die feste Zusammensetzung umfaßt, einen getrockneten Mikroorganismus, welcher Maleinsäure zu Milchsäure fermentieren kann und dabei $CO_2$ produziert, und einen festen Inhibitor der Glykolyse.

13. Zusammensetzung nach Anspruch 12, worin das Bakterium ausgewählt ist aus der Gruppe von Bakterien, welche zu der *Oenococcus*-Familie und der *Lactobacillus*-Familie gehört.

14. Zusammensetzung nach Anspruch 12, worin der Inhibitor NaF ist.

15. Zusammensetzung nach Anspruch 12, worin der Mikroorganismus gefriergetrocknete Bakterien und der Inhibitor NaF ist.

16. Zusammensetzung nach Anspruch 15, worin die Zusammensetzung etwa 2,5 g NaF pro 1000 g Bakterien umfasst.

17. Verfahren nach einer der Ansprüche 1-11, wobei das Verfahren die Verwendung einer Zusammensetzung nach einer der Ansprüche 12-16 umfasst.

## Revendications

1. Procédé pour la détermination de la quantité d'acide malique dans un jus de fruits ou un jus de fruits fermenté, ledit procédé comprenant les étapes comprenant les étapes suivantes :

   i) la mise à disposition d'un jus de fruits ou d'un jus de fruits fermenté,
   ii) la mise à disposition d'un organisme microbien test capable de fermenter l'acide malique en acide lactique et de produire de cette façon du $CO_2$,
   iii) la mise en contact dudit jus de fruits ou jus de fruits fermenté avec ledit organisme microbien test,
   iv) l'ajout d'un inhibiteur de la glycolyse,
   v) la fermentation essentiellement de tout l'acide malique, où ladite fermentation a pour résultat la production de $CO_2$ dans une quantité pouvant être mise directement en corrélation avec la quantité d'acide malique présente dans le jus de fruits ou le jus de fruits fermenté,
   vi) la détermination de la quantité de $CO_2$ produite,
   vii) la corrélation de la quantité de $CO_2$ produite à la quantité d'acide malique, et la détermination de cette façon de la quantité d'acide malique.

2. Procédé selon la revendication 1, dans lequel l'étape de détermination de la quantité de $CO_2$ produite implique :

   a) la mise en contact dudit organisme microbien test et dudit jus de fruits ou jus de fruits fermenté dans un espace confiné d'un récipient étanche à l'air ayant une première pression,
   b) l'incubation dudit organisme microbien test et du jus de fruits ou du jus de fruits fermenté pendant une durée prédéterminée,
   c) la détermination d'une seconde pression dans ledit espace confiné suivant ladite incubation, et
   d) la corrélation de ladite seconde pression à la teneur de départ de l'acide malique présent dans le jus de fruits ou le jus de fruits fermenté, et la détermination de cette façon de la quantité de $CO_2$ produite.

3. Procédé selon la revendication 1, dans lequel le procédé est employé pour contrôler un procédé de fermentation, ledit procédé de fermentation comprenant les étapes suivantes :

i) la mise à disposition d'un jus de fruits ou d'un jus de fruits fermenté,

ii) la mise à disposition d'un organisme microbien capable de fermenter l'acide malique,

iii) la définition d'un paramètre de fermentation prédéterminé,

iv) l'incubation dudit jus de fruits ou jus de fruits fermenté avec ledit organisme microbien dans des conditions permettant audit organisme microbien de fermenter au moins une partie de l'acide malique,

v) l'obtention de ladite composition liquide, après un temps d'incubation prédéterminé, un échantillon d'essai,

vi) la détermination de la quantité d'acide malique selon le procédé selon l'une quelconque des revendications 1 et 2 ; et

vii) la comparaison de la quantité d'acide malique avec ledit paramètre de fermentation prédéterminé, et

viii) le contrôle dudit procédé de fermentation en se basant sur la comparaison de la quantité dudit acide malique avec ledit paramètre de fermentation prédéterminé.

4. Procédé selon l'une quelconque des revendications 1 à 3, dans lequel le jus de fruits ou le jus de fruits fermenté est du jus de raisin ou du jus de raisin fermenté.

5. Procédé selon l'une quelconque des revendications 1 à 3, dans lequel l'inhibiteur est le NaF.

6. Procédé selon l'une quelconque des revendications 1 à 3, dans lequel l'organisme microbien est choisi dans le groupe des bactéries appartenant à la famille des *Oenococcus* et à la famille des *Lactobacillus.*

7. Procédé selon l'une quelconque des revendications 1 à 3, dans lequel l'organisme microbien test est sous forme séchée.

8. Procédé selon l'une quelconque des revendications 2 à 3, dans lequel le récipient est étanche à l'air, stable à la pression et pénétrable par une aiguille.

9. Procédé selon l'une quelconque des revendications 2 à 3, dans lequel la pression est déterminée par un manomètre comprenant une aiguille d'injection et dans lequel ladite aiguille d'injection est injectée à l'intérieur du récipient.

10. Procédé selon la revendication 2, dans lequel ladite durée prédéterminée est dans la gamme de 5 minutes à 5 heures.

11. Procédé selon la revendication 1, dans lequel l'organisme microbien test est remis en suspension dans un milieu liquide avant d'être mis en contact avec ladite composition liquide.

12. Composition solide pour la détermination de la quantité d'acide malique dans un jus de fruits ou un jus de fruits fermenté, ladite composition solide comprenant un organisme microbien séché capable de fermenter l'acide malique en acide lactique, en produisant de cette façon du $CO_2$, et un inhibiteur solide de la glycolyse.

13. Composition selon la revendication 12, dans laquelle la bactérie est choisie dans le groupe des bactéries appartenant à la famille des *Oenococcus* et à la famille des *Lactobacillus.*

14. Composition selon la revendication 12, dans laquelle l'inhibiteur est le NaF.

15. Composition selon la revendication 12, dans laquelle l'organisme microbien est une bactérie lyophilisée et l'inhibiteur est le NaF.

16. Composition selon la revendication 15, dans laquelle la composition comprend environ 2,5 g de NaF pour 1 000 g de bactéries.

17. Procédé selon l'une quelconque des revendications 1 à 11, dans lequel le procédé implique l'utilisation d'une composition selon l'une quelconque des revendications 12 à 16.

Fig. 1

## Fig. 2

## Fig. 3

Fig. 4

EP 1 490 504 B1